(19) Europäisches Patentamt
European Patent Office
Office européen des brevets

(11) EP 4 033 494 A1

(12) EUROPEAN PATENT APPLICATION

(43) Date of publication:
27.07.2022 Bulletin 2022/30

(51) International Patent Classification (IPC):
*G16H 20/60* [(2018.01)]

(21) Application number: 21152795.7

(22) Date of filing: 21.01.2021

(52) Cooperative Patent Classification (CPC):
G16H 50/30; G16H 20/60

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Koninklijke Philips N.V.
5656 AG Eindhoven (NL)

(72) Inventors:
• LOWET, Dietwig Jos Clement
5656 AE Eindhoven (NL)
• HOONHOUT, Henriette Christine Marie
5656 AE Eindhoven (NL)

(74) Representative: de Vries, Janna et al
Philips Domestic Appliances Nederland B.V.
High Tech Campus 42
5656 AE Eindhoven (NL)

(54) **PROCESSING OF RECIPE INFORMATION**

(57) A method and system are provided for processing information from a recipe to generate a summary. The summary comprises information relating to a subset of nutrients, and they are chosen based on a nutrient interest score which combines a health impact indicator (HI) and a relative quantity indicator (SR). The relative quantity indicator indicates the quantity of the nutrient relative to the quantity in a reference group of recipes. The nutrient interest score is a measure of an expected interest level in that particular nutrient.

FIG. 2

**Description**

FIELD OF THE INVENTION

This invention relates to the processing of recipe information.

BACKGROUND OF THE INVENTION

It is well known to provide advice to users in respect of the nutritional value of recipes, as well as providing recipe instructions.

When browsing for recipes, users typically check each recipe using a number of key criteria to decide whether they want to prepare the recipe or not. These criteria can be different for each user but for most users include the tastiness, the amount of effort needed (this can be a combination of the amount of time, the difficulty level, the familiarity etc.) and the health impact.

The health impact of a recipe is becoming an increasingly important criterion for many people. While the healthiness of a recipe can to a large extent be estimated based on some rules of thumb (amount of vegetables, red meat or not etc.), there is still a need to have more accurate information.

Various systems are known for addressing this issue. For example there are various apps (such as the "NutriU" app of the applicant) which provide overviews of the macro- and micro-nutrients for each recipe stored in a recipe database. This overview for example indicates the weight of each ingredient per portion, and the percentage of the recommended daily intake.

However, this information is not simple to understand and does not provide context as to whether the recipe is performing well or poorly relative to other recipes.

The number of ingredients and nutrients in a recipe is typically large so that a simple to understand summary of the health aspect of any particular recipe is difficult to achieve.

The invention is directed to this issue and aims to summarize automatically the health impact of a selected recipe in a manner which can be easily understood by a user and which captures the key information of interest.

SUMMARY OF THE INVENTION

The invention is defined by the claims.

According to examples in accordance with an aspect of the invention, there is provided a computer-implemented method for processing information from a recipe to generate a summary, comprising:

receiving a recipe or an identification of a recipe, selected by a user;
for a set of nutrients in the selected recipe and for the quantity of each nutrient in the recipe, determining a health impact indicator and a relative quantity indicator, wherein the relative quantity indicator indicates the quantity of the nutrient relative to the quantity in a reference group of recipes;
combining the health impact indicator and the relative quantity indicator to derive a nutrient interest score;
selecting a subset of the set of nutrients based on the nutrient interest score; and
generating a summary of the selected recipe comprising information relating to the selected subset of nutrients.

This method provides a summary of a recipe selected by a user, by selecting those nutrients which should be included in the summary. A nutrient interest score is derived for each nutrient of possible interest (i.e. which form the set of nutrients). This nutrient interest score is a measure of an expected interest level in that particular nutrient. In particular, it takes account of both the health impact of that nutrient (in the quantity in that it is used in the recipe) and the extent to which the amount of the nutrient is unusual. The relative quantity indicator may be considered to be a measure of surprise. Thus, the combined nutrient interest score is used to enable the information which will be of particular interest to the user to be extracted.

The recipe of interest is for example selected by a user from a database of recipes, and the reference group of recipes to be used for comparison purposes is also from the database.

Selecting a subset of the set of nutrients may be based on a simple ranking of the nutrient interest score, although other measures are possible.

The method may comprise:

selecting one or more alternative recipes, thereby forming, together with the selected recipe, a set of recipes;
determining the health impact indicator and the relative quantity indicator and selecting a subset of the set of nutrients in all recipes of the set of recipes; and

generating a summary of each recipe in the set.

The main aim of the invention is to provide engaging information about a recipe related to the health aspects of the recipe. However, with this extension to the main idea, a user can select a recipe, and the method presents alternative recipes, together with the corresponding nutrient information of interest. The user can then make a selection.

The method may comprise categorizing the selected recipe, and selecting the set of recipes with a corresponding categorization.

This categorization enables suitable alternative recipes to be selected. They may be of the same meal type (e.g. breakfast, lunch, dinner, snack) and/or of the same key ingredients (e.g. pasta, rice, meat, vegetarian) and/or the same cultural type of food (e.g. Moroccan, German).

In one approach, the reference group of recipes may be the same as the set of recipes. Thus, a selected recipe is compared with a group of recipes, and summaries are prepared for all recipes in the group. In this case, a set of possible alternative recipes is chosen, and the relative quantity indicator relates to the recipes in that set.

Alternatively, the reference group of recipes may be different to the set of recipes.

In this case, a smaller set of possible alternative recipes may be chosen to form the comparison set, whereas the relative quantity indicator relates to a larger reference group of recipes so that the relative quantity indicator relates to the more general prevalence of the nutrient in recipes.

The reference group of recipes may comprise:

all recipes in a database of recipes; or
a subset of recipes in a database to which subset the selected recipe belongs.

The relative quantity indicator thus typically relates to a larger group of recipes than the set being summarized, and it may be all recipes in the database or a subset.

The summary may comprise a textual summary comprising a set of sentences. This gives the user easily understandable information about the selected recipe (and optionally also alternative recipes in a set).

The textual summary for example explains, for the selected subset of nutrients, the nutrient quantity, the health impact of the nutrient and the relative quantity of the nutrient. This provides in a concise form all information which is likely to be of interest to the user.

Combining the health impact indicator and the relative quantity indicator may comprise a multiplication. This provides a simple way to create a measure of likely interest of the information about a nutrient.

The method may comprising receiving health information for the user, and wherein the health impact indicator is derived taking account of the health information. In this way, user health conditions may be taken into account in determining the health impact indicator. For example, for a diabetic or heart failure patient, the health impact of different nutrients will be different. This health information may also take account of allergies.

User preferences may also be taken into account (e.g. vegetarian) both in selecting the group of reference recipes (so that the recipe is being compared with suitable references) and in selecting a comparison set. The user conditions and preferences may thus be used when selecting the other recipes for the reference group (which form the reference information) and/or for the set (which form the set to be summarized).

The method may comprise obtaining the health impact indicator from a stored function of health impact versus nutrient quantity for each nutrient.

The relative quantity indicator is for example based on a mean and standard deviation of the quantity of the nutrient in the reference group of recipes. Thus, the relative quantity indicator is a statistical measure of the how unusual a particular amount of a nutrient is, compared to the other recipes in the reference group.

The invention also provides a computer program comprising computer program code which is adapted, when said program is run on a computer, to implement the method defined above.

The invention also provides a processor programmed to run the computer program defined above.

The invention also provides a system for processing information from a recipe to generate a summary, comprising an input for receiving a recipe or identification of a recipe selected by a user and the processor defined above.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows a computer-implemented method for processing information from a recipe to generate a summary;

Figure 2 shows an example of the health impact indicator for one nutrient; and

Figure 3 shows a system for processing information from a recipe to generate a summary.

## DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method and system for processing information from a recipe to generate a summary. The summary comprises information relating to a subset of nutrients, and they are chosen based on a nutrient interest score which combines a health impact indicator and a relative quantity indicator. The relative quantity indicator indicates the quantity of the nutrient relative to the quantity in a reference group of recipes. The nutrient interest score is a measure of an expected interest level in that particular nutrient.

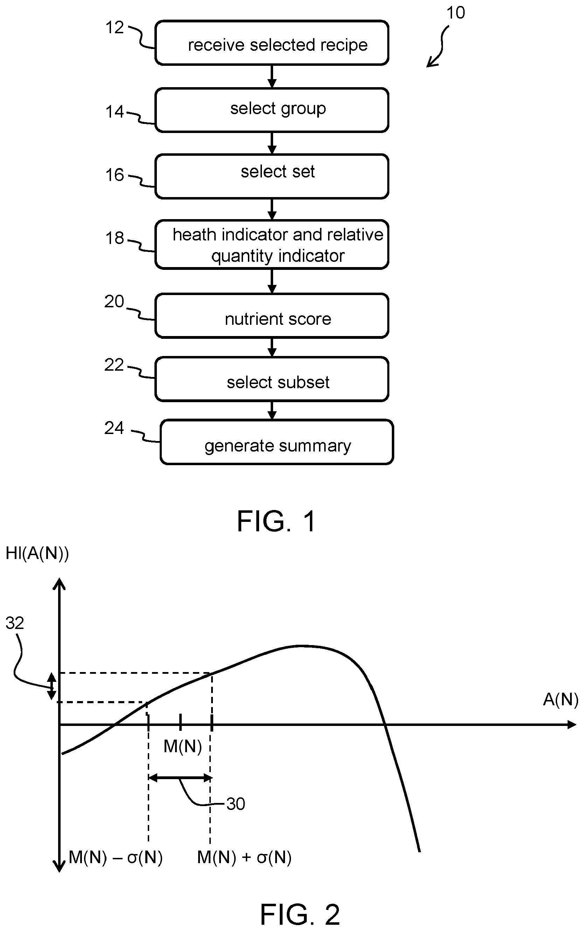

Figure 1 shows a computer-implemented method 10 for processing information from a recipe to generate a summary.

In step 12, a recipe is received (or an identification of a recipe is received) which has been selected by the user. The recipe is for example one of a database of recipes supported by the method. The user thus may simply select one of the database entries, to find out more information about the recipe and then follow it if desired. The recipe comprises ingredients and cooking instructions, in conventional manner.

The database stores information not only of the ingredients, but also identifies nutrient information for those ingredients. The nutrient information for example identifies calories, carbohydrates, fats (saturated and unsaturated), minerals, vitamins, proteins and fiber content. Each of these may be considered to be different examples of "nutrients". The nutrient information may be part of the recipe entry in the database or it may be in a separate database.

There is a set of nutrients of general interest (e.g. vitamins, minerals etc.), and the method aims to identify those nutrients from that set which are worthy of being discussed and highlighted in a summary of the particular recipe.

In step 14, a reference group of recipes is selected from the database which form a suitable reference against which the selected recipe can be compared.

This reference group of recipes may be all of the recipes in the database. Alternatively, it may be a subset of the full set of recipes in the database, and which are a suitable group against which the selected recipe can be compared.

As explained below, the purpose is to identify when a particular recipe has some nutrients in unusual amounts compared to other recipes, since such information will be of interest to the user.

In optional step 16, a set of recipes is automatically selected. This step is performed if the user wants to be presented with alternative recipe options, instead of only being presented with information about their selected recipe. The user may also or instead manually select their own set of recipes for comparison.

The set of recipes may be the same as the reference group of recipes. However, more typically, the set of recipes is a small set (e.g. 2 to 5) recipes which may be presented to the user as possible alternatives, whereas the reference group of recipes is a larger set (e.g. 10 to 500) which provides an indication of the amount of various nutrients generally found in different recipes.

The set of recipes functions as a comparison set. The user is choosing from a set of recipes, and will often want to compare recipes, The user is mostly interested in how a specific recipe of interest compares to other recipes of the same type. For this purpose, the recipes are classified so that a selected recipe can be compared with a set of relevant recipes. The classification may be based on the meal type, e.g. a breakfast, lunch, dinner or snack recipe. It may be further based on a main ingredient type (rice, pasta etc.) or meal style (e.g. nationality). For each such category, a representative set of recipes is used for the comparison when it is automatically selected by the method.

The comparison set may for example be the set of all breakfast recipes enabling statements such as *"This recipe contains a lot of vitamin D for a breakfast"*. The set may be the set of of all lunch recipes, all dinner recipes, all snacks recipes etc.

The comparison set may however be more narrowly defined, such as within dinner recipes, additionally only the pasta recipes, or within all lunch recipes only the salads, etc.

The system can also be based on user preferences. For example, the comparison set may be based on a "user recipe set": e.g. the set of recipes the user has cooked already, or has viewed within the system. The user may for example be able to select a recipe which contains the most vitamin C out of *of all the recipes they regularly cook.*

A selected recipe can also be compared to different comparison sets. Which set to use depends on configuration settings. An alternative is to select that set of recipes that produces the highest nutrient interest scores (explained below). For example, a pasta dish can be compared to other "main meals", or, more specifically, to other pasta dishes.

In step 18, for the set of nutrients generally of interest, and also taking account of the amounts present in the particular recipe, a health impact indicator and a relative quantity indicator are determined. The health impact indicator is a measure of the positive or negative health benefit and the relative quantity indicator indicates the quantity of the nutrient relative to the quantity in the reference group of recipes. The health impact indicator and relative quantity indicator are for example normalized to a per-portion measure.

In step 20, the health impact indicator and the relative quantity indicator are combined to derive a nutrient interest score. A nutrient interest score is thus derived for each nutrient of possible interest (i.e. which form the set of nutrients generally of interest). This nutrient interest score is a measure of an expected interest level in that particular nutrient. In particular, it takes account of both the health impact of that nutrient (in the quantity in that it is present in the recipe) and the extent to which the amount of the nutrient is unusual. The relative quantity indicator may be considered to be a measure of surprise. The nutrient interest score identifies which nutrients have information which will be of particular interest to the user, i.e. be particularly surprising.

In step 22, a subset of the set of nutrients is selected based on the nutrient interest score. Selecting the subset of the set of nutrients may be based on a simple ranking of the nutrient interest score, although other measures are possible.

A summary of the selected recipe is then prepared in step 24 comprising information relating to the selected subset of nutrients.

When a comparison set of recipes has been selected (manually or automatically), the health impact indicator and the relative quantity indicator are also determined for the nutrients in those other recipes, and a subset of the set of nutrients are selected in all recipes of the set of recipes. A summary of each recipe in the set is in that case generated in step 24. A user can thus select a recipe, and the method presents alternative recipes, together with the corresponding nutrient information of interest. The user can then make a selection.

An example of a possible way to derive a nutrient interest score will now be explained in more detail. The nutrient interest score is an indication of a likely level of interest in the particular nutrient and it is based on a combination of the health impact indicator and the relative quantity indicator.

The health impact indicator may be expressed as $HI(A(N))$ and it represents how great is the impact of this amount $A(N)$ of nutrient $(N)$ on health. This health impact can be both negative and positive and the function $A(N)$ might be different for different nutrients.

For each nutrient N, a custom-made health impact indicator function, $HI(A(N))$ is used. This can be done with varying degrees of sophistication. In a most simple approach, the health impact indicator function could be approximated with a positive constant for nutrients that people typically lack (e.g. long fibers) and a negative constant for nutrients for which people typically consume too much (e.g. saturated fats, added sugar).

However, many nutrients are healthy in small quantities, have a certain optimal quantity for a meal and above this quantity start having less optimal impact, and even above a certain quantity have a negative (or even poisonous) effect on the body.

Figure 2 shows an example of the health impact indicator $(HI(A(N)))$ for one nutrient. A positive $HI(A(N))$ represents a positive health benefit and a negative $HI(A(N))$ represents a negative health benefit.

The range 30 of values of A(N) represents the typical spread of the amount of nutrient N in the recipes of the reference group, i.e. the general range (within one standard deviation each side of the mean M(N)). The range 32 represents the spread in health impact for this general range of nutrient amount.

The exact curve will be different for each nutrient. Therefore the function may be tailored to each nutrient separately based on knowledge from literature.

The health impact can also be person dependent. For example, the impact of sugar or salt is much stronger on a person with diabetes or hypertension, respectively. The health impact indicator functions can therefor be personalized, e.g. the function of sugar for people with diabetes will be different than for healthy people.

The relative quantity indicator may be expressed as $SR(A(N))$. It is a measure of a surprise rating and indicates by how much does this nutrient differs from the average. The surprise rating measures by how much a nutrient health fact related to this recipe differs from other (potentially similar) recipes. For example, it may express how much the amount of long fibers in this particular recipe differs from other similar recipes.

For a recipe R, the following values can be defined:

$A(N)$: Amount of a certain nutrient N in the recipe R;

$\mu(N)$ : average amount of a certain nutrient N in the reference group of recipes;

$\sigma(N)$: standard deviation of a certain nutrient N in the reference group of recipes;

c: a baseline value (a baseline level of expected surprise rating about the amount of a particular nutrient).

The relative quantity indicator may then be defined as:

$$SR(N, R) \sim c + \frac{|A(N) - \mu(N)|}{\sigma(N)}$$

The relative quantity indicator is in this example based on a mean and standard deviation of the quantity of the nutrient in the reference group of recipes. Thus, the relative quantity indicator is a statistical measure of the how unusual a particular amount of a nutrient is, compared to the other recipes in the reference group.

This formula assumes a Gaussian distribution for nutrient N in the various recipes. However, for a certain nutrient, the distribution could be quite different from a Gaussian distribution. If the actual distribution differs too much, a different distribution can be used e.g. a Chi distribution.

Any suitable statistical measure may be used which represents the degree by which the sample i.e. A(N) departs from the mean (or median or other expected level).

This formula captures the surprising part of the nutritional fact. This formula depends on comparing the current recipe with a reference group of recipes as explained above.

As also explained above, the reference group may be the set of all the recipes in the database or it could be a subset to which the current recipe belongs.

In a most basic approach, the nutrient interest score, for use in determining which nutrients are selected for providing health information in the summary of the recipe, is given by a multiplicative relation, which basically states that if either the health impact indicator and the relative quantity indicator (surprise level) are zero, the nutrient interest score is zero and if both HI and SR are large, the nutrient interest score is large.

$$I(N, A(N), R)) = |HI(N, A(N))| * SR(A(N), R)$$

Note again that the health impact indicator can be both negative or positive. A large nutrient interest score based on a negative health impact indicator may be of interest as a warning.

However more complex, functions might be used.

The nutrients are then ranked according to the nutrient interest score. The leading ones are then selected to form the basis of a recipe summary.

For each nutrient within the selected set, a sentence may then be formed describing the health impact and the surprising information relating to the relative quantity. The source or sources of the nutrient of interest can also be explained (e.g. broccoli or rice). The creation of this sentence can be based on selecting a suitable sentence from a predefined set of sentences. The summary may be based on a combination of multiple different sentences.

A first example summary for a *"salmon with broccoli"* recipe may be *"This recipe (i.e., salmon with broccoli) contains 4 grams of omega-3 fatty acids per portion, this is 2 times more than the average main dish. Omega-3 fatty acids play several important roles in your body. They have anti-inflammatory effects and are an essential component of your brain and eyes. The broccoli in the recipe provides a large amount vitamin K, 220 micro-gram. Vitamin K is important for bone metabolism and regulating blood calcium levels. "*

A second example summary for a *"cake-with-little"* sugar recipe may be *"This recipe contains only 10 grams of added sugar. This is 5 times less than a typical cake. In the Western world, diets contain too much sugar which can increase the risk of developing diabetes. Daily intake of added sugar should ideally be less than 37 grams for a healthy male adult. "*

The summary may thus comprise a textual summary comprising a set of sentences. This gives the user easily understandable information about the selected recipe (and optionally alternative recipes in a set). The summary conveys, for the selected subset of nutrients, general information about the actual nutrient quantity, the health impact of the nutrient and the relative quantity of the nutrient.

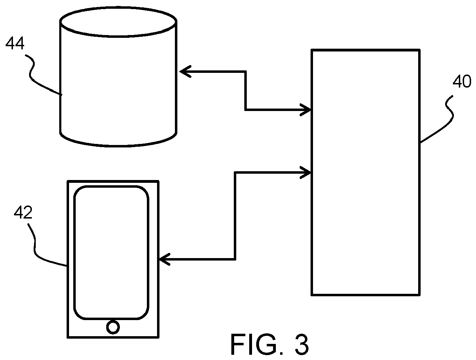

Figure 3 shows a system for processing information from a recipe to generate a summary. The system comprises a processor 40 which runs a computer program to implement the described above.

The processor accesses a database 44 of recipes. This database may be stored locally with the processor or it may be remote from the processor. The user has a user interface device such as a mobile phone 42 on which a suitable app is loaded. The user uses the mobile phone to select a recipe, and to provide user information to the processor 40. This user information is for example the user identity, their preferences, such as dietary preferences, and any medical conditions. The processor thus has an input receiving a recipe or identification of a recipe selected by a user and it generates the summary as explained above.

The processor is for example a remote server with which the mobile phone communicates and the database may be part of that remote server.

The summary may be adapted to the health profile or eating pattern of the user: For example, people following a vegan diet run a high risk of deficiencies in certain nutrients, e.g. B12 (cobalamin), and hence the amount of this nutrient in a recipe is much more important than for non-vegetarian user. Similarly, the summary can be adapted for people with certain health risks: e.g. elderly, pregnant women,

The example above is limited to health aspects that relate to nutrients of the recipe. However, the method may be extended to also include other aspect such as .the cooking process.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims. (optional)

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems. (optional)

If the term "adapted to" is used in the claims or description, it is noted the term "adapted to" is intended to be equivalent to the term "configured to".

Any reference signs in the claims should not be construed as limiting the scope.

**Claims**

1. A computer-implemented method (10) for processing information from a recipe to generate a summary, comprising:

   (12) receiving a recipe or an identification of a recipe, selected by a user;
   (18) for a set of nutrients in the selected recipe and for the quantity of each nutrient in the recipe, determining a health impact indicator (HI) and a relative quantity indicator (SR), wherein the relative quantity indicator indicates the quantity of the nutrient relative to the quantity in a reference group of recipes;
   (20) combining the health impact indicator and the relative quantity indicator to derive a nutrient interest score;
   (22) selecting a subset of the set of nutrients based on the nutrient interest score; and
   (24) generating a summary of the selected recipe comprising information relating to the selected subset of nutrients.

2. The method of claim 1, comprising:

   (16) selecting one or more alternative recipes, thereby forming, together with the selected recipe, a set of recipes;
   (18) determining the health impact indicator (HI) and the relative quantity indicator (SR) and selecting a subset of the set of nutrients in all recipes of the set of recipes; and
   (24) generating a summary of each recipe in the set.

3. The method of claim 2, comprising categorizing the selected recipe, and selecting the set of recipes with a corresponding categorization.

4. The method of claim 2 or 3, wherein the reference group of recipes is the same as the set of recipes.

5. The method of claim 2 or 3, wherein the reference group of recipes is different to the set of recipes.

6. The method of claim 5, wherein the reference group of recipes comprises:

   all recipes in a database of recipes; or
   a subset of recipes in a database to which subset the selected recipe belongs.

7. The method of any one of claims 1 to 6, wherein the summary comprises a textual summary comprising a set of sentences.

8. The method of claim 7, wherein the textual summary explains, for the selected subset of nutrients, the nutrient quantity, the health impact of the nutrient and the relative quantity of the nutrient.

9. The method of any one of claim 1 to 8, wherein combining the health impact indicator and the relative quantity indicator comprises a multiplication.

10. The method of any one of claim 1 to 9, comprising receiving health information for the user, and wherein the health impact indicator is derived taking account of the health information.

11. The method of any one of claim 1 to 10, comprising obtaining the health impact indicator from a stored function of health impact versus nutrient quantity for each nutrient.

12. The method of any one of claim 1 to 11, comprising obtaining the relative quantity indicator based on a mean and standard deviation of the quantity of the nutrient in the reference group of recipes.

13. A computer program comprising computer program code which is adapted, when said program is run on a computer, to implement the method of any one of claims 1 to 12.

14. A processor programmed to run the computer program of claim 13.

15. A system for processing information from a recipe to generate a summary, comprising:

    an input for receiving a recipe or identification of a recipe selected by a user; and
    the processor of claim 14.

FIG. 1

FIG. 2

FIG. 3

Europäisches
Patentamt

European
Patent Office

Office européen
des brevets

**EUROPEAN SEARCH REPORT**

Application Number

EP 21 15 2795

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/216982 A1 (BENNETT GEORGE B [US] ET AL) 22 August 2013 (2013-08-22)<br>* paragraphs [0006], [0007]; figures 1A,1B,4,24,25 *<br>* paragraphs [0152] - [0157] *<br>----- | 1-15 | INV.<br>G16H20/60 |

TECHNICAL FIELDS
SEARCHED      (IPC)

G16H

The present search report has been drawn up for all claims

| Place of search | Date of completion of the search | Examiner |
|---|---|---|
| Munich | 19 May 2021 | Michalski, Stéphane |

EPO FORM 1503 03.82 (P04C01)

## ANNEX TO THE EUROPEAN SEARCH REPORT
## ON EUROPEAN PATENT APPLICATION NO.

EP 21 15 2795

This annex lists the patent family members relating to the patent documents cited in the above-mentioned European search report.
The members are as contained in the European Patent Office EDP file on
The European Patent Office is in no way liable for these particulars which are merely given for the purpose of information.

19-05-2021

| Patent document cited in search report | Publication date | Patent family member(s) | Publication date |
|---|---|---|---|
| US 2013216982 A1 | 22-08-2013 | AU 2013101802 A4 | 16-05-2019 |
| | | AU 2013221301 A1 | 04-09-2014 |
| | | AU 2018203143 A1 | 24-05-2018 |
| | | AU 2020223691 A1 | 10-09-2020 |
| | | CA 2864741 A1 | 22-08-2013 |
| | | CN 104303185 A | 21-01-2015 |
| | | CN 107845414 A | 27-03-2018 |
| | | CN 107863137 A | 30-03-2018 |
| | | EP 2815339 A2 | 24-12-2014 |
| | | HK 1205569 A1 | 18-12-2015 |
| | | HK 1206450 A1 | 08-01-2016 |
| | | HK 1252934 A1 | 06-06-2019 |
| | | HK 1252959 A1 | 06-06-2019 |
| | | JP 6412429 B2 | 24-10-2018 |
| | | JP 6725619 B2 | 22-07-2020 |
| | | JP 2015510646 A | 09-04-2015 |
| | | JP 2018200730 A | 20-12-2018 |
| | | JP 2020187756 A | 19-11-2020 |
| | | US 2013216982 A1 | 22-08-2013 |
| | | US 2015194071 A1 | 09-07-2015 |
| | | WO 2013123416 A2 | 22-08-2013 |

EPO FORM P0459

For more details about this annex : see Official Journal of the European Patent Office, No. 12/82